# EUROPÄISCHE PATENTSCHRIFT

(11) **EP 2 718 003 B1**
(45) Veröffentlichungstag und Bekanntmachung des Hinweises auf die Patenterteilung: **06.03.2019**
(21) Anmeldenummer: 12723860.8
(22) Anmeldetag: 23.05.2012
(51) Int. Cl.: B01J 13/02, A61K 8/11

(54) **KERN-HÜLLE-PARTIKEL MIT EINEM HOHEN GEHALT AN GLYCERIN, IHRE HERSTELLUNG UND VERWENDUNG**
CORE-SHEATH PARTICLES HAVING A HIGH CONTENT OF GLYCEROL, PRODUCTION AND USE THEREOF
PARTICULES DE TYPE NOYAU-ENVELOPPE AYANT UNE TENEUR ÉLEVÉE EN GLYCÉRINE, LEUR FABRICATION ET LEUR UTILISATION

(30) Priorität: 09.06.2011 DE 102011077298
(43) Veröffentlichungstag der Anmeldung: 16.04.2014
(73) Patentinhaber: Evonik Degussa GmbH, 45128 Essen (DE)
(72) Erfinder: RIEDEMANN, Heike, 63776 Mömbris (DE); MÜNZENBERG, Jörg, 63456 Hanau (DE)
(74) Vertreter: Evonik Patent Association
(86) Internationale Anmeldenummer: PCT/EP2012/059563
(87) Internationale Veröffentlichungsnummer: WO 2012/168073

(56) Entgegenhaltungen:
- EP-A1- 1 787 957
- EP-A1- 1 787 958
- WO-A1-2009/120526
- US-A1- 2005 276 831
- US-A1- 2007 202 063
- US-A1- 2008 118 568
- US-A1- 2010 008 870

## Beschreibung

Die Erfindung betrifft Kern-Hülle-Partikel, deren Hülle aggregierte, hydrophobierte Siliciumdioxid-Partikel und deren Kern eine flüssige Phase enthalten. Den Hauptbestandteil der flüssigen Phase bildet Glycerin. Die Erfindung umfasst weiterhin die Herstellung dieser Kern-Hülle-Partikel sowie ihre Verwendung zu kosmetischen Zwecken.

Das Verfahren zur Herstellung von so genanntem "trockenen Wasser" ("Dry Water"), bei dem Flüssigkeitströpfchen von hydrophobierten, pyrogen hergestellten Siliciumdioxid-Partikeln umhüllt werden, ist aus DE 1467023 bekannt. Durch die Hülle aus Siliciumdioxid-Partikeln werden die Flüssigkeitströpfchen an der Koaleszenz gehindert, und es entsteht ein pulverförmiges Gemisch. Durch Verreiben lässt sich das Wasser wieder freisetzen.

EP 0855177 A2 offenbart einen aufhellenden Puder, der unter anderem 0,1 - 7 Gew.-% hydrophobierte Kieselsäure, 5 - 40 Gew.-% mehrwertige Alkohole und 50 - 94 Gew.-% Wasser enthält. Ein höherer Anteil an mehrwertigen Alkoholen wird als unvorteilhaft für das Hautgefühl beschrieben. Für eine Mischung mit einem Wassergehalt von weniger als 50 % wird eine unvollständige Verflüssigung vorausgesagt.

Der Stand der Technik beschreibt die Einkapselung einer flüssigen Phase durch hydrophobierte Kieselsäurepartikel, wobei die flüssige Phase als Hauptbestandteil Wasser enthält. Diesen Formulierungen können nur Stoffe zugesetzt werden, die in der Wasserphase löslich bzw. mit der Wasserphase mischbar sind. Darüber hinaus ist bekannt, dass "Dry-Water"-Formulierungen sensibel auf die Zugabe von Zusatzstoffen reagieren, so dass unter Umständen keine Kern-Hülle-Struktur entsteht. Daraus folgt, dass "Dry Water"-Formulierungen sowohl in Bezug auf die verwendbaren Zusatzstoffe als auch deren Konzentration Einschränkungen unterliegen.

"Dry Water"-Formulierungen weisen zudem häufig eine eingeschränkte Stabilität auf. Bei längerer Lagerung, insbesondere bei erhöhten Temperaturen, verdampft das in der Hülle eingeschlossene Wasser. Dadurch verändert sich mit der Zeit die Zusammensetzung des Produkts. Bei geschlossener Lagerung eines "Dry-Water" kondensiert das verdampfte Wasser beim Abkühlen an der Behälterwand und sammelt sich am Boden des Behälters, so dass die "Dry Water"-Formulierung nicht mehr verwendbar ist.

Die technische Aufgabe der Erfindung ist es daher, Kern-Hülle-Partikel bereitzustellen, die diese Nachteile nicht aufweisen.

Ein Gegenstand der Erfindung sind Kern-Hülle-Partikel, deren Hülle aggregierte, hydrophobierte Siliciumdioxid-Partikel und deren Kern eine flüssige Phase enthalten, und bei denen das Verhältnis von aggregierten, hydrophobierten Siliciumdioxid-Partikeln zur flüssigen Phase, bezogen auf das Gesamtgewicht der Kern-Hülle-Partikel, 2:98 bis 40:60 beträgt, wobei in der flüssigen Phase 60 - 100 Gew.-% Glycerin enthalten sind.

Kern-Hülle-Partikel bedeutet hierbei, dass Flüssigkeitströpfchen von den aggregierten, hydrophobierten Siliciumdioxid-Partikeln umgeben werden, so dass Partikel mit einer Teilchengröße von 0,1 - 50 µm, in der Regel von 1 - 10 µm, gebildet werden. Die Flüssigkeit im Kern kann sowohl eine Lösung, eine Emulsion oder Bestandteil einer Dispersion sein. Bevorzugt ist sie eine Lösung. Durch Reibung, Druck oder auch Wärme kann die Hülle aufgebrochen werden, so dass die Flüssigkeit freigesetzt wird und für den gewünschten Verwendungszweck zur Verfügung steht.

Unter Aggregaten sind fest, beispielsweise über Sinterhälse, miteinander verbundene Primärpartikel zu verstehen. Die Aggregate ihrerseits können sich zu Agglomeraten zusammenlagern, in denen die Aggregate nur lose miteinander verbunden sind. Agglomerate können durch Einbringen von geringen Scherenergien wieder gespalten werden.

Die Art der aggregierten, hydrophobierten Siliciumdioxid-Partikel ist nicht beschränkt solange sichergestellt ist, dass bei ihrer Zugabe zu der flüssigen Phase Kern-Hülle-Partikel entstehen. Die aggregierten, hydrophobierten Siliciumdioxid-Partikel können bevorzugt silanisiert sein. Zur Silanisierung können Halogensilane, Alkoxysilane, Silazane und/oder Siloxane eingesetzt werden.

Insbesondere können als Halogensilane die folgenden Stoffe eingesetzt werden:
- Halogenorganosilane des Typs X₃Si(CₙH2ₙ₊₁) mit X = Cl, Br und n = 1 - 20,
- Halogenorganosilane des Typs X₂(R')Si(CₙH₂ₙ₊₁) mit X = Cl, Br und R' = Alkyl, n = 1 - 20
- Halogenorganosilane des Typs X(R')₂Si(CₙH₂ₙ₊₁) mit X = Cl, Br, R' = Alkyl, n = 1 - 20
- Halogenorganosilane des Typs X₃Si(CH2)ₘ-R' mit X = Cl, Br, m = 0 - 20, R' = Alkyl, Aryl (zum Beispiel -C₆H₅), -C₄F₉, -OCF₂-CHF-CF₃, -C₆F₁₃, -O-CF₂-CHF₂, -NH₂, -N₃, -SCN, -CH=CH₂, -OOC(CH₃)C=CH₂, -OCH₂-CH(O)CH₂, -NH-COO-CH₃, -NH-COO-CH₂-CH₃, -NH-(CH₂)₃Si(OR)₃,-Sx-(CH₂)₃Si(OR)₃,
- Halogenorganosilane des Typs (R)X₂Si(CH₂)ₘ-R' mit X = Cl, Br, R = Alkyl, m = 0 - 20, R' = Alkyl, Aryl (zum Beispiel -C₆H₅), -C₄F₉, -OCF₂-CHF-CF₃, -C₆F₁₃, -O-CF₂-CHF₂, -NH₂, -N₃, -SCN, -CH=CH₂, - OOC(CH₃)C = CH₂, -OCH₂-CH(O)CH₂, -NH-COO-CH₃, -NH-COO-CH₂-CH₃, -NH-(CH₂)₃Si(OR)₃, -NH-COO-CH₃, -NH-COO-CH₂-CH₃, -NH-(CH₂)₃Si(OR)₃,-Sₓ-(CH₂)₃Si(OR)₃
- Halogenorganosilane des Typs (R)₂X Si(CH₂)ₘ-R' mit X = Cl, Br, R = Alkyl, m = 0 - 20, R' = Alkyl, Aryl (zum Beispiel -C₆H₅), -C₄F₉, -OCF₂-CHF-CF₃, -C₆F₁₃, -O-CF₂-CHF₂, -NH₂, -N₃, -SCN, -CH=CH₂, - OOC(CH₃)C = CH₂, -OCH₂-CH(O)CH₂, -NH-COO-CH₃, -NH-COO-CH₂-CH₃, -NH-(CH₂)₃Si(OR)₃,-Sx-(CH₂)₃Si(OR)₃

Insbesondere können als Alkoxysilane die folgenden Stoffe eingesetzt werden:
- Organosilane des Typs (RO)₃Si(CₙH₂ₙ₊₁) mit R = Alkyl, n=1 - 20
- Organosilane des Typs R'ₓ(RO)_{y}Si(CₙH₂ₙ₊₁) mit R = Alkyl, R' = Alkyl, n = 1 - 20, x+y = 3, x = 1,2, y = 1,2
- Organosilane des Typs (RO)₃Si(CH₂)ₘ-R' mit R = Alkyl, m = 0 - 20, R' = Alkyl, Aryl (zum Beispiel -C₆H₅), -C₄F₉, OCF₂-CHF-CF₃, -C₆F₁₃, -O-CF₂-CHF₂, -NH₂, -N₃, -SCN, -CH=CH₂, -OOC(CH₃)C = CH₂, -OCH₂-CH(O)CH₂, -NH-COO-CH₃, -NH-COO-CH₂-CH₃, -NH-(CH₂)₃Si(OR)₃, -Sₓ-(CH₂)₃Si(OR)₃
- Organosilane des Typs (R")ₓ(RO)_{y}Si(CH₂)ₘ-R' mit R" = Alkyl, x+y = 2, x = 1,2, y = 1,2, m = 0 - 20, R' = Alkyl, Aryl (zum Beispiel -C₆H₅), -C₄F₉, - OCF₂-CHF-CF₃, -C₆F₁₃, -O-CF₂-CHF₂, -NH₂, -N₃, -SCN, -CH=CH₂, - OOC(CH₃)C = CH₂, -OCH₂-CH(O)CH₂, NH-COO-CH₃, -NH-COO-CH₂-CH₃, -NH-(CH₂)₃Si(OR)₃, -Sₓ-(CH₂)₃Si(OR)₃

Bevorzugt kann man als Silanisierungsmittel Trimethoxyoctylsilan [(CH₃O)3-Si-C₈H₁₇] (zum Beispiel Dynasylan® OCTMO, Evonik Degussa) einsetzen.

Insbesondere können als Silazane die folgenden Stoffe eingesetzt werden:
- Silazane des Typs R'R₂Si-NH-SiR₂R' mit R, R' = Alkyl, Vinyl, Aryl sowie zum Beispiel Hexamethyldisilazan (zum Beispiel Dynasylan® HMDS, Evonik Degussa).

Insbesondere können als Siloxane die folgenden Stoffe eingesetzt werden:
- Cyclische Polysiloxane des Typs D 3, D 4, D 5 und ihre Homologen, wobei unter D 3, D 4, D 5 cyclische Polysiloxane mit 3, 4 oder 5 Einheiten des Typs -O-Si(CH₃)₂ verstanden wird, zum Beispiel Octamethylcyclotetrasiloxan = D 4.
- Polysiloxane bzw. Silikonöle des Typs Y-O-[(R¹R²SiO)ₘ-(R³R⁴SiO)ₙ]ᵤ-Y, mit R¹, R², R³, R⁴ = unabhängig voneinander Alkyl, wie CₙH₂ₙ₊₁,
   n = 1-20; Aryl, wie Phenylradikale und substitutierte Phenylradikale, (CH₂)ₙ-NH₂, H
   Y = CH₃, H, CₙH₂ₙ₊₁, n = 2 - 20; Si(CH₃)₃, Si(CH₃)₂H, Si(CH₃)₂OH, Si(CH₃)₂(OCH₃), Si(CH₃)₂(CₙH₂ₙ₊₁), n = 2-20
   m = 0,1,2,3,..., 100000,
   n = 0,1,2,3,..., 100000,
   u = 0,1,2,3,..., 100000.

Die Silanisierung kann man durchführen, indem man hydrophile Siliciumdioxid-Partikel mit dem Silanisierungsmittel, das gegebenenfalls in einem organischen Lösungsmittel, wie zum Beispiel Ethanol, gelöst sein kann, besprüht und das Gemisch anschließend bei einer Temperatur von 105 bis 400 °C über einen Zeitraum von 1 bis 6 h thermisch behandelt.

Bevorzugt können Siliciumdioxid-Partikel pyrogener Herkunft eingesetzt werden. Pyrogen umfasst dabei solche Partikel, die durch Flammenoxidation oder Flammenhydrolyse aus geeigneten Siliciumverbindungen erhältlich sind. In der Regel wird Siliciumtetrachlorid in einer Flamme aus Wasserstoff und Sauerstoff zu Siliciumdioxid hydrolysiert.

Als Maß für die Hydrophobie wird in der Regel die Methanolbenetzbarkeit bestimmt. Bevorzugt weisen die aggregierten, hydrophobierten Siliciumdioxid-Partikel eine Methanolbenetzbarkeit von mindestens 35 Vol.-% Methanol auf, bevorzugt sind Werte von 38 bis 75 Vol.-% Methanol. Bei der Bestimmung der Methanolbenetzbarkeit werden in transparente Zentrifugenröhrchen jeweils 0,2 g ± 0,005 g aggregierte, hydrophobierte Siliciumdioxid-Partikel eingewogen. Es werden zu jeder Einwaage 8,0 ml eines Methanol/Wasser Gemisches mit jeweils 10, 20, 30, 40, 50, 60, 70 und 80 Vol.-% Methanol zugegeben. Die verschlossenen Röhrchen werden 30 Sekunden geschüttelt und anschließend 5 Minuten bei 2500 U/min zentrifugiert. Die Sedimentvolumina werden abgelesen, in Prozent umgerechnet und graphisch gegen den Methanolgehalt in Vol.-% aufgetragen. Der Wendepunkt der Kurve entspricht der Methanolbenetzbarkeit.

Bevorzugt können die mit Octamethylcyclotetrasiloxan, Polydimethylsiloxan, Octylsilan und/oder Hexamethyldisilazan hydrophobierten Siliciumdioxid-Partikel eingesetzt werden, wobei Hexamethyldisilazan besonders bevorzugt ist.

Die spezifische Oberfläche der aggregierten, hydrophobierten Siliciumdioxid-Partikel ist nicht beschränkt. Bevorzugt enthalten die erfindungsgemäßen Kern-Hülle-Partikel solche Siliciumdioxid-Partikel mit einer spezifischen Oberfläche von 80 bis 300 m²/g und besonders bevorzugt solche von 100 bis 250 m²/g, ganz besonders bevorzugt beträgt die spezifische Oberfläche 220 ± 25 m²/g. Die BET-Oberfläche wird nach DIN 66131 bestimmt.

Beispiele für kommerziell erhältliche aggregierte, hydrophobierte Siliciumdioxidpartikel sind AEROSIL® R 202, AEROSIL® R 805, AEROSIL® R 812, AEROSIL® R 812 S, AEROSIL® R 972, alle von Evonik Degussa. Die Eigenschaften dieser Siliciumdioxidpartikel sind in Tabelle 1 zu finden.

Bezogen auf das Gesamtgewicht der erfindungsgemäßen Kern-Hülle-Partikel beträgt der Gehalt an aggregierten, hydrophobierten Siliciumdioxid-Partikeln 2 - 40 Gew.-%, bevorzugt sind 2 - 20 Gew.-%, ganz besonders bevorzugt sind 2-15 Gew.-%.

Die erfindungsgemäßen Kern-Hülle-Partikel enthalten aggregierte, hydrophobierte Siliciumdioxid-Partikel und Glycerin, wobei Glycerin mit einem Anteil von 60 - 100 Gew.-% in der flüssigen Phase enthalten ist. Vorzugsweise ist Glycerin mit einem Gewichtsanteil von 75 - 99,5 % in der flüssigen Phase enthalten, besonders bevorzugt ist ein Anteil von 85 - 95 Gew.-%. In einer besonderen Ausführungsform besteht die flüssige Phase vollständig aus Glycerin.

Die erfindungsgemäßen Kern-Hülle-Partikel können neben Glycerin bis zu 40 Gew.-% Wasser in der flüssigen Phase enthalten, bevorzugt sind bis zu 20 Gew.-%, besonders bevorzugt sind bis zu 10 Gew.-%. In einer besonderen Ausführungsform enthält die flüssige Phase kein Wasser.

Darüber hinaus können zusätzlich kosmetische Hilfsstoffe und/oder Wirkstoffe in der flüssigen Phase enthalten sein, die der Fachmann auf dem Gebiet "Dry Water" aus dem Stand der Technik kennt. Diese Stoffe können den folgenden Gruppen angehören: UV-Lichtschutzfilter, Farbstoffe und Pigmente, Deo- und Antitranspirant-Wirkstoffe, Biogene Stoffe, Insektenrepellent-Wirkstoffe, Hydrotrope, Antischuppenwirkstoffe, Bleich- oder Hautaufhellungsmittel sowie Selbstbräuner, Konservierungsmittel, Tenside/Emulgatoren, Parfümöle und Pflanzenextrakte, Wirkstoffe, Filmbildner, Öle, Emollients und anorganische Salze.

Der Anteil an kosmetisch relevanten Hilfsstoffen und Wirkstoffen kann zusammen bis zu 25 Gew.-%, bezogen auf die flüssige Phase, betragen. Bevorzugt sind Gewichtsanteile von unter 15 Gew.-%, besonders bevorzugt sind 0,1 -10 Gew.-%, jeweils bezogen auf die flüssige Phase.

An anorganischen Salzen können insbesondere die Halogenide, Carbonate, Hydrogencarbonate, Borate, Sulfate, Phosphate, Hydrogenphosphate, Nitrate, Nitrite, Silikate oder Ammoniumsalze der Alkali- und Erdalkaliionen verwendet werden. Ganz besonders bevorzugt ist wenigstens eines der folgenden Salze: NaCl, KCl, MgSO₄, MgCl2, CaCl2, NaHCO₃. Salze werden mit einer Gesamtkonzentration von bis zu 5 Gew.-% in wässriger Lösung, bezogen auf das Gesamtgewicht der Lösung, eingesetzt, besonders bevorzugt ist eine Gesamtkonzentration von 0,1 - 3,5 Gew.-%.

Besonders geeignet sind darüber hinaus folgende Stoffe: Aluminiumchlorohydrat (Locron® P), Vitamin E-acetat, Parfümöle, Pigmente, Vinylpyrrolidon-Vinylacetat-Copolymer (Kollidon® VP/VA-Copolymer), Aloe Vera, sowie als Emollient TEGOSOFT® GMC 6, ABIL® B 8843 oder ABIL® B 8852, alle drei Evonik Goldschmidt.

Die Zugabe von Hilfsstoffen und/oder Wirkstoffen kann die Oberflächenspannung der flüssigen Phase beeinflussen. Der Wert der Oberflächenspannung der flüssigen Phase der erfindungsgemäßen Kern-Hülle-Partikel sollte bei 25 °C in der Regel 55 - 75 mN/m betragen. Bevorzugt beträgt die Oberflächenspannung 60 - 67 mN/m, besonders bevorzugt sind Werte von 62 - 65 mN/m, jeweils bei 25 °C. Die Oberflächenspannung wird nach der Ringmethode (ISO 304) bestimmt.

Ein weiterer Gegenstand der Erfindung ist ein Verfahren zur Herstellung der Kern-Hülle-Partikel, bei dem die Bestandteile der flüssigen Phase zunächst vorgelegt und gegebenenfalls homogenisiert werden, anschließend die agggregierten, hydrophobierten Siliciumdioxid-Partikel zugegeben werden und die Mischung geschert wird.

Ein weiterer Gegenstand der Erfindung ist ein die Kern-Hülle-Partikel enthaltendes Haarstylingpulver. Unter einem Haarstylingpulver ist davei ein Pulver zur zeitweiligen Verformung menschlichen Haares zu verstehen.

Die Erfindung umfasst weiterhin die Verwendung der Kern-Hülle-Partikel zu kosmetischen Zwecken.

### Beispiele und Formulierungen:

Die Versuche wurden alle mit DAB-Glycerin mit einem Glycerin-Gehalt von 99,5 % durchgeführt. Die eingesetzten aggregierten, hydrophobierten Siliciumdioxid-Partikel stammen alle von Evonik Degussa.

### Allgemeine Durchführung:

Die erfindungsgemäßen Kern-Hülle-Partikel werden erhalten, indem man die Bestandteile der flüssigen Phase zunächst vorlegt und gegebenenfalls homogenisiert, anschließend die aggregierten, hydrophobierten Siliciumdioxid-Partikel zugibt und die Mischung schert. Das erhaltene Produkt wird in Kunststoffbehälter abgefüllt. Die Konsistenz des Produktes wird visuell beurteilt.

Die eingesetzten Mengenangaben und Scherparameter können Tabelle 2 entnommen werden.

### Beispiel 1:

Es werden 98 g Glycerin in einem 500-mL-Edelstahlbecher des Dissolvers DISPERMAT® CA-40 M1 (Firma VMA-Getzmann GmbH, Scheibendurchmesser 5 cm) vorgelegt. Anschließend werden 2 g AEROSIL® R 812 S zugegeben. Der Mischbecher wird mit einem Deckel verschlossen. Die Scherung wird für 2 Minuten bei 3000 U/min durchgeführt. Es entstehen pulverförmige, frei fließende Kern-Hülle-Partikel.

Beispiele 2 bis 14 werden analog hergestellt.
Aus den Beispielen 2 bis 12 werden jeweils pulverförmige, frei fließende Kern-Hülle-Partikel erhalten, während aus den Vergleichsbeispielen 13 und 14 jeweils eine Creme erhalten wird.
Die Eigenschaften der Kern-Hülle-Partikel werden visuell beurteilt.

**Tabelle 1: Verwendete Siliciumdioxid-Partikel**

| **Hydrophobiertes Siliciumdioxid** | **Hydrophobisierungs mittel** | **Spez. Oberfläche (BET) m²/g** | **Methanolbenetzbar keit [Vol.-% Methanol]** | **Kohlenstoffgehalt (Gew.-%)** |
|---|---|---|---|---|
| AEROSIL® R 812 S | Hexamethyldisilazan | 220 ± 25 | 52-60 | 3,0 - 4,0 |
| AEROSIL® R 805 | Octylsilan | 150 ± 25 | 45 | 4,5 - 6,5 |
| AEROSIL® R 202 | Polydimethylsiloxan | 100 ± 20 | 70 | 3,5 - 5,0 |
| AEROSIL® R 972 | Dimethyldichlorsilan | 110 ± 20 | 38 | 0,6 - 1,2 |

**Tabelle 2: Zusammensetzung und Eigenschaften der beispielhaften Formulierungen**

| **Beispiel** | **AEROSIL® R** | **[g** | **Glycerin [g]** | **Wasser [g]** | **Zusatz, [g]** | **Verfahren [U/min]/[min]** | **Eigenschaft** |
|---|---|---|---|---|---|---|---|
| 1 | **812 S** | 2 | 98 | - | - | 3000/2 | Pulver, frei fließend |
| 2 | **812 S** | 5 | 95 | - | - | 3000/3 | Pulver, frei fließend |
| 3 | **972** | 5 | 95 | - | - | 3000/3 | Pulver, frei fließend |
| 4 | **805** | 5 | 95 | - | - | 3000/3 | Pulver, frei fließend |
| 5 | **202** | 5 | 95 | - | - | 5000/5 | Pulver, frei fließend |
| 6 | **812 S** | 5 | 66,5 | 28,5 | - | 6000/6 | Pulver, frei fließend |
| 7 | **812 S** | 5 | 90 | - | Pigmentgemisch¹ 5 | 3000/2 | Pulver, frei fließend |
| 8 | **812 S** | 5 | 76 | - | Locron® P, 19 | 3000/3 | Pulver, frei fließend |
| 9 | **812 S** | 10 | 87,6 | - | PEG-6, 2,7 | 3000/2 | Pulver, frei fließend |
| 10 | **812 S** | 5 | 94,05 | - | VP/VA-Copolymer, 0,95 | 3000/2 | Pulver, frei fließend |
| 11 | **812 S** | 5 | 66,5 | - | Salzwasser (3,5 %ig), 28,5 | 3000/3 | Pulver, frei fließend |
| 12 | **812 S** | 10 | 89 | - | Bienenwachs, 1 | 4000/3 | Pulver, frei fließend |
| 13 | **812 S** | 5 | - | - | Ethylenglykol, 95 | 3000/3 | weiße Creme |
| 14 | **812 S** | 5 | - | - | Polyethylen glykol, 95 | 2500/5 | klare Creme |

| | | | | | | | |
|---|---|---|---|---|---|---|---|
| ¹ W1802 Pigment Jaune Covasil S, INCI: Cl 77492, Trimethoxycaprylylsilan 45 Gew.-%, W3801 Pigment Rouge Covasil S, INCI: Cl 77491, Trimethoxycaprylylsilan 15 Gew.-%, W9814 Pigment Noir Covasil S, INCI: Cl 77499, Trimethoxycaprylylsilan 10 Gew.-%, Oxyde de Titane STD Covasil, INCI: Cl 77891, Trimethoxycaprylylsilan 30 Gew.-%, alle von Sensient Cosmetic Technologies | | | | | | | |

## Patentansprüche

1. Kern-Hülle-Partikel, deren Hülle aggregierte, hydrophobierte Siliciumdioxid-Partikel und deren Kern eine flüssige Phase enthalten, und bei denen das Verhältnis von aggregierten, hydrophobierten Siliciumdioxid-Partikeln zur flüssigen Phase, bezogen auf das Gesamtgewicht der Partikel, 2:98 bis 40:60 beträgt, **dadurch gekennzeichnet, dass** in der flüssigen Phase 60 - 100 Gew.-% Glycerin enthalten sind.

2. Kern-Hülle-Partikel nach Anspruch 1, **dadurch gekennzeichnet, dass** neben Glycerin bis zu 40 Gew.-% Wasser in der flüssigen Phase enthalten sind.

3. Kern-Hülle-Partikel nach den Ansprüchen 1 oder 2, **dadurch gekennzeichnet, dass** in der flüssigen Phase zusätzlich kosmetische Hilfsstoffe enthalten sind.

4. Kern-Hülle-Partikel nach den Ansprüchen 1 oder 2, **dadurch gekennzeichnet, dass** in der flüssigen Phase zusätzlich kosmetische Wirkstoffe enthalten sind.

5. Kern-Hülle-Partikel nach den Ansprüchen 3 oder 4, **dadurch gekennzeichnet, dass** der Gewichtsanteil der kosmetischen Hilfsstoffe und Wirkstoffe zusammen bis zu 25 Gew.-%, bezogen auf die flüssige Phase, beträgt.

6. Kern-Hülle-Partikel nach den Ansprüchen 1 bis 5, **dadurch gekennzeichnet, dass** die Oberflächenspannung der flüssigen Phase bei 25 °C 55 - 75 mN/m beträgt.

7. Verfahren zur Herstellung von Kern-Hülle-Partikeln gemäß den Ansprüchen 1 bis 6, **dadurch gekennzeichnet, dass** die Bestandteile der flüssigen Phase zunächst vorgelegt und gegebenenfalls homogenisiert werden, anschließend die aggregierten, hydrophobierten Siliciumdioxid-Partikel zugegeben werden und die Mischung geschert wird.

8. Haarstylingpulver enthaltend die Kern-Hülle-Partikel nach den Ansprüchen 1 bis 6.

9. Verwendung der Kern-Hülle-Partikel nach den Ansprüchen 1 bis 6 zu kosmetischen Zwecken.

## Claims

1. Core-shell particles, the shell of which comprises aggregated, hydrophobicized silicon dioxide particles, and the core of which comprises a liquid phase, and in which the ratio of aggregated, hydrophobicized silicon dioxide particles to the liquid phase, based on the total weight of the particles, is 2:98 to 40:60, **characterized in that** 60-100% by weight of glycerol are present in the liquid phase.

2. Core-shell particles according to Claim 1, **characterized in that**, besides glycerol, up to 40% by weight of water are present in the liquid phase.

3. Core-shell particles according to Claim 1 or 2, **characterized in that** cosmetic auxiliaries are additionally present in the liquid phase.

4. Core-shell particles according to Claim 1 or 2, **characterized in that** cosmetic active ingredients are additionally present in the liquid phase.

5. Core-shell particles according to Claim 3 or 4, **characterized in that** the weight fraction of the cosmetic auxiliaries and active ingredients together is up to 25% by weight, based on the liquid phase.

6. Core-shell particles according to Claims 1 to 5, **characterized in that** the surface tension of the liquid phase at 25°C is 55-75 mN/m.

7. Process for producing core-shell particles according to Claims 1 to 6, **characterized in that** the constituents of the liquid phase are firstly introduced as initial charge and are optionally homogenized, then the aggregated, hydrophobicized silicon dioxide particles are added and the mixture is sheared.

8. Hair-styling powder comprising the core-shell particles according to Claims 1 to 6.

9. Use of the core-shell particles according to Claims 1 to 6 for cosmetic purposes.

## Revendications

1. Particules noyau-enveloppe, dont l'enveloppe contient des particules de dioxyde de silicium hydrophobées agrégées et dont le noyau contient une phase liquide, et dans lesquelles le rapport entre les particules de dioxyde de silicium hydrophobées agrégées et la phase liquide, par rapport au poids total des particules, est de 2:98 à 40:60, **caractérisées en ce que** la phase liquide contient 60 à 100 % en poids de glycérine.

2. Particules noyau-enveloppe selon la revendication 1, **caractérisées en ce que** la phase liquide contient jusqu'à 40 % en poids d'eau en plus de la glycérine.

3. Particules noyau-enveloppe selon les revendications 1 ou 2, **caractérisées en ce que** des adjuvants cosmétiques sont en outre contenus dans la phase liquide.

4. Particules noyau-enveloppe selon les revendications 1 ou 2, **caractérisées en ce que** des agents actifs cosmétiques sont en outre contenus dans la phase liquide.

5. Particules noyau-enveloppe selon les revendications 3 ou 4, **caractérisées en ce que** la proportion en poids des adjuvants et des agents actifs cosmétiques ensemble est de jusqu'à 25 % en poids, par rapport à la phase liquide.

6. Particules noyau-enveloppe selon les revendications 1 à 5, **caractérisées en ce que** la tension de surface de la phase liquide à 25 °C est de 55 à 75 mN/m.

7. Procédé de fabrication de particules noyau-enveloppe selon les revendications 1 à 6, **caractérisé en ce que** les constituants de la phase liquide sont tout d'abord chargés et éventuellement homogénéisés, puis les particules de dioxyde de silicium hydrophobées agrégées sont ajoutées et le mélange est cisaillé.

8. Poudre pour le coiffage des cheveux, contenant les particules noyau-enveloppe selon les revendications 1 à 6.

9. Utilisation des particules noyau-enveloppe selon les revendications 1 à 6 à des fins cosmétiques.
